# EUROPEAN PATENT APPLICATION

(11) **EP 3 907 212 A1**
(43) Date of publication of application: **10.11.2021**
(21) Application number: 20382372.9
(22) Date of filing: 06.05.2020
(51) Int. Cl.: C07C 51/295, C07C 51/31, C07C 67/475

(54) **METAL-FREE METHOD FOR OXIDATIVE CLEAVAGE OF VIC-DIOLS TO CARBOXYLIC ACIDS**

(71) Applicant: Consejo Superior de Investigaciones Científicas (CSIC), 28006 Madrid (ES); Universidad De Zaragoza, 50009 Zaragoza (ES)
(72) Inventor: FRAILE DOLADO, José María, 50009 Zaragoza (Zaragoza) (ES); DORADO HORRILLO, Vicente, 50009 Zaragoza (Zaragoza) (ES); HERRERÍAS LARRIPA, Clara Isabel, 50009 Zaragoza (Zaragoza) (ES)
(74) Representative: Pons

(57) **Abstract**

The invention relates to a simple and efficient metal-free method for oxidative cleavage of *vic*-diols, in particular those derived from fatty esters, to the corresponding carboxylic acids, using a cheap, safe and environmentally friendly oxidant under mild conditions wherein oxone® is used as an oxidant and a halide as a catalyst in presence of the accurate organic solvent.

## Description

The invention relates to a simple and efficient metal-free method for oxidative cleavage of *vic*-diols, in particular those derived from fatty esters, to the corresponding carboxylic acids, using a cheap, safe and environmentally friendly oxidant under mild conditions wherein oxone® is used as an oxidant and a halide as a catalyst in presence of the accurate organic solvent.

### BACKGROUND ART

The oxidative cleavage of 1,2-diols is a very useful synthetic organic reaction, whose research, in aspects such as the finding of more sustainable methods or the scope of the reaction, still deserves an active interest. One example is the cleavage of diols derived from fatty acids, to produce shorter acids and diacids, such as pelargonic (nonanoic) and azelaic (nonanodioic) acids from 9,10-dihydroxystearate, considered as a safe alternative for the risky ozonolysis (Santacesaria et al., Ind. Eng. Chem. Res., 2000, 39, 2766-2771).

In some cases, the oxidative cleavage has been coupled to the dihydroxylation reaction, for example by addition of Co(OAc) as a second catalyst in the reaction mixture containing H₂O₂ and H₂WO₄, or by using a stoichiometric oxidant, such as NaIO₄ or NaOCl. The oxidative cleavage of 9,10-dihydroxystearate with O₂ and NaOH has been reported with supported gold nanoparticles as catalyst (Kulik et al. Green Chem., 2014, 16, 1799-1806). The corresponding aldehydes (nonanal and 9-oxononanoic acid) can be obtained by cleavage of 9,10-dihydroxystearate with H₂O₂ catalysed by WO₃/MCM-41, or by cleavage of 9,10-epoxystearate with H₂O₂ catalysed by WO₃/mesoporous-SnO₂ in a release-and-capture catalytic system (Lu et al. Green Chem., 2019, 21, 560-566). Hence, the oxidation methods with environmentally friendly oxidants require the use of heavy metal catalysts, whereas the metal-free methods use rather expensive or environmentally unfriendly oxidants.

Recently, other methods for oxidative cleavage of different *vic*-diols have been reported. Some of them use molecular oxygen as an oxidant with either homogeneous catalysts, such as AgOTf in basic (NaOMe) medium, or heterogeneous catalysts, for example a mixture of Pt/C and V₂O₅, or a Mn layered mixed oxide. Other oxidants have been used in combination with metal catalysts, such as NMO with perruthenate, or DMSO with MoO₂Cl₂. However, metal-free methods are scarce, including the use of hypervalent iodine oxidants and nitroxyl radicals as catalysts, and the O₂/NaO^{t}Bu system.

As can be seen, the methods described suffer from the need for costly reagents or the poor performance with aliphatic diols. Hence, the development of new metal-free methods of oxidative cleavage of diols, using cheap and environmentally friendly oxidants, and suitable for aliphatic diols is still a problem to be solved.

### SUMMARY OF THE INVENTION

The present invention discloses a novel method of transformation of *vic*-diols under environmentally friendly conditions, using oxone® (2KHSO₅·KHSO₄·K₂SO₄) to cleavage the C-C bond and to get carboxylic acids. Particularly, the reaction has been studied using syn-9,10-dihydroxystearate as starting material, and obtaining pelargonic acid and azelaic acid monomethyl ester as the only final products with a conversion higher than 90%.

When an alternative method for oxidation using aqueous H₂O₂ as an oxidant and a mixture of KBr (40 mol%) and p-toluenesulfonic acid (40 mol%) as catalyst in a biphasic dichloromethane/water medium was applied to methyl syn-9,10-dihydroxystearate, the conversion was much lower (55%) and the observed products were methyl 9,10-dioxostearate (diketone) and methyl 9(10)-hydroxy-10(9)-oxostearate (hydroxyketone), whereas the cleavage of the 1,2-diol products were not detected (see Example 3).

Therefore, the present invention relates to a process of cleavage of 1,2-diols, in particular those derived from fatty esters, to the corresponding carboxylic acids, under mild conditions with the presence of oxone®/ halide.

A first aspect of the present invention related to process for producing carboxylic acids which comprises a cleavage reaction of 1,2-diols, using 2KHSO₅·KHSO₄·K₂SO₄ as an oxidant and a halide of formula MXₙ, wherein M is an alkaline or alkaline earth metal, X is a halogen and n is 1 or 2, as a catalyst, in presence of a solvent, at a temperature that ranges between 20 to 60 °C, according to the following scheme: 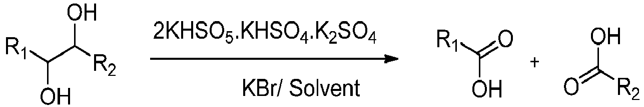 wherein R₁ and R₂ are independently selected from an optionally substituted C₁-C₂₀ alkyl group, optionally substituted C₆-C₂₀ aryl group, optionally substituted C₅-C₁₄ heteroaryl group, and -RCOOR', wherein R and R' are independently an optionally substituted C₁-C₂₀ alkyl group.

In a preferred embodiment the amount of 2KHSO₅·KHSO₄·K₂SO₄ ranges between 2 and 10 equivalents; more preferably 3 to 5 equivalents are used.

The role of the halide MXₙ was shown to be essential, as no conversion was observed without this catalyst under the same conditions. Therefore, in another preferred embodiment the amount of this halide ranges between 5 and 100 mol% equivalents; more preferably 20 to 40 mol% equivalents are used.

In another preferred embodiment the metal M in the halide is selected from an alkaline (Li, Na, K, Rb, Cs) or an alkaline earth (Mg, Ca, Sr, Ba).

In another preferred embodiment the halogen X in the alkali halide is selected from bromide or chlorine.

In another preferred embodiment the halide is KBr.

In another preferred embodiment the solvent is a mixture of an organic polar solvent and water, wherein the organic polar solvent is selected from an alcohol (e.g. tert-butanol), an ether (e.g. tetrahydrofuran), an ester (e.g. ethyl acetate), a nitrile (e.g. acetonitrile), a nitrocompound (e.g. nitromethane), a haloalkane (e.g. dichloromethane), an amide (e.g. dimethyl formamide), a sulfoxide (e.g. dimethylsulfoxide), a ketone (e.g. acetone) or a mixture thereof; more preferably the solvent in the cleavage reaction is acetonitrile/water or tert-butanol/water.

In another preferred embodiment the reaction takes place at a temperature that ranges between 10 to 80 °C, more preferably from 20 to 40°C.

In another preferred embodiment R₁ is an optionally substituted C₁-C₂₀ alkyl group; even more preferred is a C₁-C₂₀ alkyl group selected from methyl, ethyl, n-propyl, 1-methylethyl (isopropyl), n-butyl, 1,1-dimethylethyl (t-butyl), n-pentyl, n-heptyl, n-octyl, n-nonyl, n- n-decyl, n-undecyl, and n-dodecyl.

In another preferred embodiment R₁ and R₂ form together an optionally substituted C₃-C₁₂ cycloalkyl group; even more preferred is a C₃-C₁₂ cycloalkyl group selected from cyclopropyl, cyclobutyl, cyclopentyl, and cyclohexyl.

In another preferred embodiment R₁ and R₂ are independently an optionally substituted C₆-C₂₀ aryl group; even more preferred is a C₆-C₂₀ aryl group selected from phenyl, naphthyl, tetrahydronapthyl, indanyl, and biphenyl.

In another preferred embodiment R₂ is -RCOOR', wherein R and R' an optionally substituted C₁-C₂₀ alkyl group, selected from methyl, ethyl, n-propyl, 1-methylethyl (isopropyl), n-butyl, 1,1-dimethylethyl (t-butyl), n-pentyl, n-heptyl, n-octyl, n-nonyl, n-n-decyl, n-undecyl, and n-dodecyl.

The term 'alkyl' as used herein refers to a straight or branched hydrocarbon chain radical consisting solely of carbon and hydrogen atoms, containing no unsaturation, having from one to twenty carbon atoms, and which is attached to the rest of the molecule by a single bond.

The term "cycloalkyl" as used herein refers to a non-aromatic mono or multicyclic ring system of about 3 to 12 carbon atoms such as cyclopropyl, cyclobutyl, cyclopentyl, and cyclohexyl. Examples of multicyclic cycloalkyl groups include perhydronapththyl, bridged cyclic groups, e.g. adamantyl and norbornyl groups, and spirobicyclic groups, e.g., spiro[4,4]non-2-yl.

The term "aryl" as used herein refers to aromatic radicals having in the range of 6 up to 20 carbon atoms.

The term "heteroaryl" as used herein refers to an optionally substituted 5 to 14 members aromatic ring having one or more heteroatoms selected from N, O, and S as ring atoms. The heteroaryl may be a mono-, bi- or tricyclic ring system. Examples of such "heterocyclic ring" or "heteroaryl" radicals include, but are not limited to, oxazolyl, thiazolyl, imidazolyl, pyrrolyl, furanyl, pyridinyl, pyrimidinyl, pyrazinyl, benzofuranyl, indolyl, benzothiazolyl, benzoxazolyl, carbazolyl, quinolyl , isoquinolyl, azetidinyl, acridinyl, benzodioxolyl, benzodioxanyl, benzofuranyl, carbazolyl, cinnolinyl, dioxolanyl, indolizinyl, naphthyridinyl, perhydroazepinyl, phenazinyl, phenothiazinyl, phenoxazinyl, phthalazinyl, pteridinyl, purinyl, quinazolinyl, quinoxalinyl, tetrazoyl, tetrahydroisoquinolyl, piperidinyl, piperazinyl, 2-oxopiperazinyl, 2-oxopiperidinyl, 2-oxopyrrolidinyl, 2-oxoazepinyl, azepinyl, 4-piperidonyl, pyrrolidinyl, pyridazinyl, oxazolinyl, oxazolidinyl, triazolyl, indanyl, isoxazolyl, isoxazolidinyl, morpholinyl, thiazolinyl, thiazolidinyl, isothiazolyl, quinuclidinyl, isothiazolidinyl, isoindolyl, indolinyl, isoindolinyl, octahydroindolyl, octahydroisoindolyl, decahydroisoquinolyl, benzimidazolyl, thiadiazolyl, benzopyranyl, tetrahydrofuryl, tetrahydropyranyl, thienyl, benzothienyl, thiamorpholinyl, thiamorpholinyl sulfoxide, thiamorpholinyl sulfone, dioxaphospholanyl, oxadiazolyl, chromanyl, and isochromanyl.

The term "substituted" unless otherwise specified, refers to substitution with any one or any combination of the following substituents and may be the same or different which one or more are selected from the groups such as hydrogen, hydroxy, halogen, carboxyl, cyano, nitro, oxo (=O), thio(=S), substituted or unsubstituted alkyl, substituted or unsubstituted alkoxy, substituted or unsubstituted alkenyl, substituted or unsubstituted alkynyl, substituted or unsubstituted cycloalkyl, substituted or unsubstituted cycloalkenyl, substituted or unsubstituted cycloalkylalkyl, substituted or unsubstituted cycloalkenylalkyl, substituted or unsubstituted heterocyclyl, substituted or unsubstituted heterocyclylalkyl, substituted or unsubstituted aryl, substituted or unsubstituted arylalkyl, substituted or unsubstituted heteroaryl, substituted or unsubstituted heteroarylalkyl, -COOR', -C(O)R', -C(S)R', -C(O)NR'R", -C(O)ONR'R", -NR'R", -NR'CONR'R", -N(R')SOR", -N(R')SO₂R", -(=N-N(R')R"), - NR'C(O)OR", -NR'R", -NR'C(O)R"-, -NR'C(S)R" -NR'C(S)NR"R"', -SONR'R"-, -SO₂NR'R"-, -OR', - OR'C(O)NR"R"', -OR'C(O)OR"-, -OC(O)R', -OC(O)NR'R",- R'NR"C(O)R"', -R'OR", - R'C(O)OR", -R'C(O)NR"R"', -R'C(O)R", -R'OC(O)R", -SR', -SOR', -SO₂R', -ONO₂ wherein R', R" and R'" in each of the above groups can be hydrogen, hydroxy, halogen, carboxyl, cyano, nitro, oxo (=O), thio(=S), imino (=NR'), substituted or unsubstituted alkyl, substituted or unsubstituted alkoxy, substituted or unsubstituted alkenyl, substituted or unsubstituted alkynyl, substituted or unsubstituted cycloalkyl, substituted or unsubstituted cycloalkenyl, substituted or unsubstituted cycloalkylalkyl, substituted or unsubstituted cycloalkenylalkyl, substituted or unsubstituted heterocyclyl, substituted or unsubstituted heterocyclylalkyl, substituted or unsubstituted aryl, substituted or unsubstituted arylalkyl, substituted or unsubstituted heteroaryl, substituted or unsubstituted heteroarylalkyl, or any two of R', R" and R"' may be joined to form a substituted or unsubstituted saturated or unsaturated 3-10 membered ring, which may optionally include heteroatoms which may be the same or different and are selected from O, NR^{X} or S or form oxo (=O), thio(=S) or imino (=NR'). Substitution or the combinations of substituents envisioned by this invention are preferably those that result in the formation of a stable or chemically feasible compound. The term stable as used herein refers to the compounds or the structure that are not substantially altered when subjected to conditions to allow for their isolation, production, detection and preferably their recovery, purification and incorporation into a pharmaceutical composition.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings are provided by way of illustration and are not intended to be limiting of the present invention.

### BRIEF DESCRIPTION OF THE DRAWINGS

**Fig. 1****.** Gas chromatogram of the oxidative cleavage of methyl syn-9,10-dihydroxystearate with oxone/KBr.
**Fig. 2****.** ¹H-NMR spectrum of the mixture of methyl nonanoate and dimethyl azelate obtained by esterification of the crude of the oxidative cleavage.

### EXAMPLES

### General Methods

The reactions were monitored using an Agilent 7890A equipped with a FID detector. The capillary column was a ZB-5HT Inferno: 30 m × 0.25 mm × 0.25 µm. Helium was used as carrier gas at 17 psi in column head. Injector temperature: 280 °C. Detector temperature: 250 °C. Oven program: 70°C (4 min), 25°C min-1 to 150°C (0 min) and 5°C min-1 to 250°C (15 min).

### Retention times:

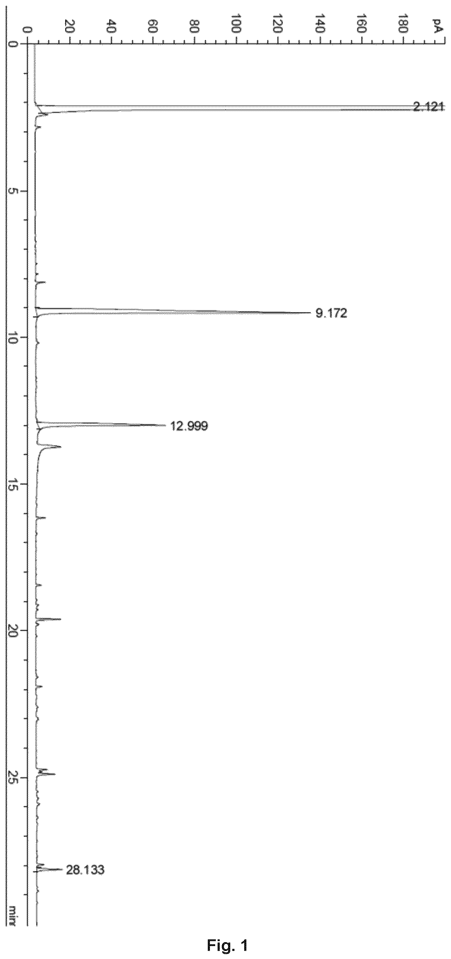

Cleavage of methyl 9,10-dihydroxystearates (Fig. 1): nonanoic acid, 9.2 min; azelaic acid monomethyl ester, 13.0 min; methyl syn-9,10-dihydroxystearate, 28.1 min; methyl anti-9,10-dihydroxystearate, 28.3 min.

Other detected products: methyl 9,10-dioxostearate, 24.8 min; methyl 9(10)-hydroxy-10(9)-oxostearate, 26.7 min.

Cleavage of methyl syn-13,14-dihydroxydocosanoate: nonanoic acid, 9.2 min; tridecanedioic acid monomethyl ester, 19.5 min; methyl syn-13,14-dihydroxydocosanoate, 38.6 min.

Cleavage of simple diols: trans-cyclohexane-1,2-diol, 7.2 min; benzoic acid, 8.6 min; adipic acid, 10.5 min; meso-hydrobenzoin, 16.8 min.

### Example 1. Oxidative cleavage of methyl syn-9,10-dihydroxystearate in the presence of Oxone®/KBr in ^{t}BuOH/H₂O

To a solution of methyl syn-9,10-dihydroxystearate (165.3 mg, 0.5 mmol) in 4 mL ^{t}BuOH/H₂O (3:1 v:v), were added 12 mg of KBr (0.1 mmol, 20 mol%) and 614.7 mg of Oxone® (2.0 mmol) and the mixture was stirred at room temperature for 24 h. The reaction crude was diluted with distilled water (15 mL) and the products were extracted with ethyl acetate (3 × 10 mL). The combined organic phases were washed with brine (10 mL), dried with anhydrous MgSO4, and filtered. The solvents were evaporated under reduced pressure to get a mixture of nonanoic (pelargonic) acid and nonanodioic (azelaic) acid monomethyl ester in 1:1 molar ratio (92% yield).

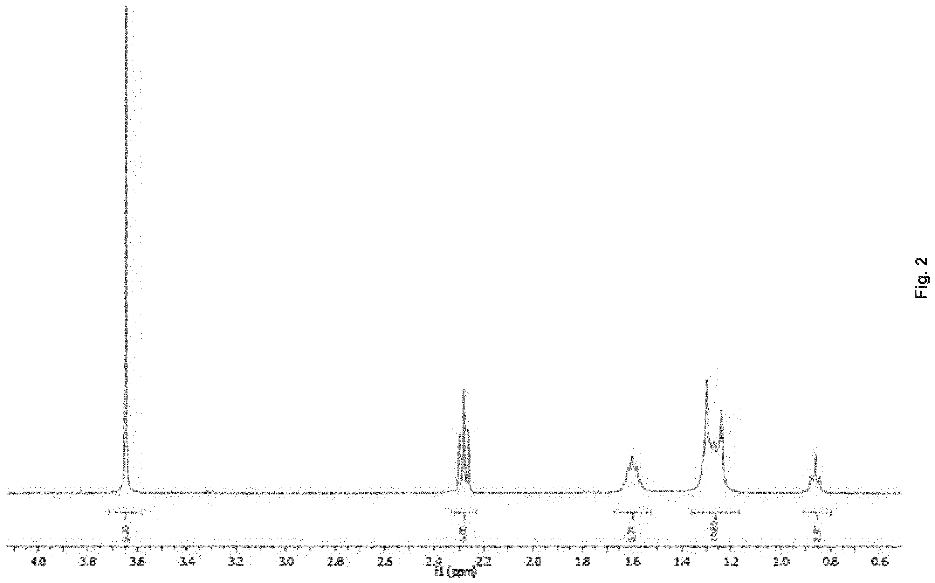

The NMR analysis was carried out after esterification. To a solution of the mixture of carboxylic acids (150 mg) in methanol (3 mL), concentrated sulfuric acid (2 drops) was added. The solution was heated at 65°C overnight. The acid was neutralized with saturated NaHCO3 aqueous solution and the products were extracted with ethyl acetate (15 mL). The organic phase was washed with water (2 × 15 mL), dried with anhydrous MgSO4, and filtered. The organic solvents were evaporated under reduced pressure.
¹H-RMN (CDCl3, δ ppm, 400 MHz): 3.64 (s, 9H), 2.28 (t, 6H, J=8 Hz), 1.67-1.53 (m, 6H), 1.36-1.22 (m, 16H), 0.86 (t, 3H, J=8 Hz) (Fig. 2).

### Example 2. Oxidative cleavage of methyl syn-9,10-dihydroxystearate in the presence of Oxone®/KBr in MeCN/H₂O

Example 1 was repeated but using 10 mL MeCN/H₂O (9:1 v:v) as solvent instead of ^{t}BuOH/H₂O (3:1 v:v). The mixture of nonanoic (pelargonic) acid and nonanodioic (azelaic) acid monomethyl ester (1:1 molar ratio) was obtained with 89% yield.

### Example 3. Oxidative cleavage of methyl syn-9,10-dihydroxystearate in the presence of Oxone® in MeCN/H₂O

Example 2 was repeated but without adding KBr to the reaction mixture. No conversion was detected after 24 h.

### Example 4. Oxidative cleavage of methyl syn-9,10-dihydroxystearate in the presence of H₂O₂/KBr/pTosOH

To a solution of methyl syn-9,10-dihydroxystearate (165.3 mg, 0.5 mmol) in 1 mL CH₂Cl₂/H₂O (9:1 v:v), were added 24 mg of KBr (0.2 mmol, 40 mol%), 34,4 mg of p-toluenesulfonic acid (0.2 mmol, 40 mol%) and 68 µL of 60% H₂O₂ (1.5 mmol) and the mixture was stirred at room temperature for 24 h. The reaction crude was diluted with distilled water (15 mL) and the products were extracted with dichloromethane (3 × 10 mL). The combined organic phases were dried with anhydrous MgSO₄, and filtered. The solvent was evaporated under reduced pressure to get a mixture of unconverted methyl syn-9,10-dihydroxystearate (45%), methyl 9(10)-hydroxy-10(9)-oxostearate (47%) and methyl 9,10-dioxostearate (8%).

### Example 5. Oxidative cleavage of methyl anti-9,10-dihydroxystearate in the presence of Oxone®/KBr in ^{t}BuOH/H₂O

Example 1 was repeated but using the anti-isomer as starting material. The mixture of nonanoic (pelargonic) acid and nonanodioic (azelaic) acid monomethyl ester (1:1 molar ratio) was obtained with 90% yield.

### Example 6. Oxidative cleavage of methyl syn-13,14-dihydroxybehenate in the presence of Oxone®/KBr in MeCN/H₂O

Example 2 was repeated but using methyl syn-13,14-dihydroxybehenate (docosanoate) as starting material. The mixture of nonanoic (pelargonic) acid and tridecanodioic acid monomethyl ester (1:1 molar ratio) was obtained with 85% yield.

### Example 7. Oxidative cleavage of trans-cyclohexane-1,2-diol in the presence of Oxone®/KBr in MeCN/H₂O

Example 2 was repeated but using trans-cyclohexane-1,2-diol as starting material. Adipic acid was obtained with 70% yield.

### Example 8. Oxidative cleavage of meso-hydrobenzoin in the presence of Oxone®/KBr in ^{t}BuOH/H₂O

Example 1 was repeated but using meso-hydrobenzoin as starting material. Benzoic acid was obtained with 65% yield.

## Claims

1. A process for producing carboxylic acids which comprises a cleavage reaction of 1,2-diols, using 2KHSO₅·KHSO₄·K₂SO₄ as an oxidant and a halide of formula MXₙ , wherein M is an alkaline or alkaline earth metal, X is a halogen and n is 1 or 2, as a catalyst, in presence of a solvent, at a temperature that ranges between 20 to 60 °C, according to the following scheme: 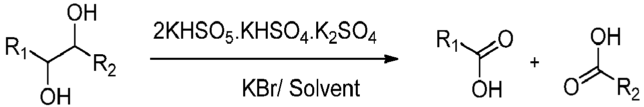 Wherein R₁ and R₂ are independently selected from an optionally substituted C₁-C₂₀ alkyl group, optionally substituted C₆-C₂₀ aryl group, optionally substituted C₅-C₁₄ heteroaryl group, and -RCOOR', wherein R and R' are independently an optionally substituted C₁-C₂₀ alkyl group.

2. Process according to claim 1, wherein the amount of 2KHSO₅·KHSO₄·K₂SO₄ ranges between 3 and 5 equivalents.

3. Process according to any preceding claim, wherein the amount of halide ranges between 20 and 40 mol% equivalents.

4. Process according to any preceding claim, wherein the metal M in the halide is selected from an alkaline or an alkaline earth.

5. Process according to any preceding claim, wherein the halogen X in the halide is selected from bromide or chlorine.

6. Process according to any preceding claim, wherein the halide is KBr

7. Process according to any preceding claim, wherein the solvent is from a mixture of an organic polar solvent and water.

8. Process according to claim 7, wherein the organic polar solvent is selected from tert-butanol, tetrahydrofuran, ethyl acetate, acetonitrile, nitromethane, dichloromethane, dimethyl formamide, dimethylsulfoxide, and acetone, or a mixture thereof.

9. Process according claim 8, wherein the solvent in the cleavage reaction is acetonitrile/water or tert-butanol/water.

10. Process according to any preceding claim, wherein the temperature ranges between 10 to 80 °C.

11. Process according to claim 10, wherein the temperature ranges from 20 to 40°C.

12. Process according to any preceding claim, wherein R₁ is an optionally substituted C₁-C₂₀ alkyl group, selected from methyl, ethyl, n-propyl, 1-methylethyl (isopropyl), n-butyl, 1,1-dimethylethyl (t-butyl), n-pentyl, n-heptyl, n-octyl, n-nonyl, n- n-decyl, n-undecyl, and n-dodecyl

13. Process according to any claims 1 to 9, wherein R₁ and R₂ form together an optionally substituted C₃-C₁₂ cycloalkyl group selected from cyclopropyl, cyclobutyl, cyclopentyl, and cyclohexyl.

14. Process according to any claims 1 to 9, wherein R₁ and R₂ are independently an optionally substituted C₆-C₂₀ aryl group selected from phenyl, naphthyl, tetrahydronapthyl, indanyl, and biphenyl.

15. Process according to any preceding claim, wherein R₂ is -RCOOR', wherein R and R' an optionally substituted C₁-C₂₀ alkyl group, selected from methyl, ethyl, n-propyl, 1-methylethyl (isopropyl), n-butyl, 1,1-dimethylethyl (t-butyl), n-pentyl, n-heptyl, n-octyl, n-nonyl, n- n-decyl, n-undecyl, and n-dodecyl.
